# EUROPÄISCHE PATENTSCHRIFT

(11) **EP 3 811 894 B1**
(45) Veröffentlichungstag und Bekanntmachung des Hinweises auf die Patenterteilung: **20.08.2025**
(21) Anmeldenummer: 20157543.8
(22) Anmeldetag: 15.02.2020
(51) Int. Cl.: A61B 6/40, A61B 46/10

(54) **BEFESTIGUNGSVORRICHTUNG ZUR BEFESTIGUNG EINES ABDECKMITTELS AN EINEM ARBEITS- ODER UNTERSUCHUNGSGERÄT**
FASTENING DEVICE FOR FIXING A COVERING ELEMENT TO A WORK OR EXAMINATION DEVICE
DISPOSITIF DE FIXATION PERMETTANT DE FIXER UN AGENT DE COUVERTURE SUR UN APPAREIL DE TRAVAIL OU D'EXAMEN

(30) Priorität: 24.10.2019 DE 202019105921 U
(43) Veröffentlichungstag der Anmeldung: 28.04.2021
(73) Patentinhaber: Bahr, Ulrich, 72290 Lossburg (DE)
(72) Erfinder: Bahr, Ulrich, 72290 Lossburg (DE)
(74) Vertreter: RPK Patenanwälte Reinhardt und Kaufmann Partnerschaft mbB (Pforzheim)

(56) Entgegenhaltungen:
- WO-A1-2018/086736
- DE-A1- 1 958 905
- US-A1- 2013 025 605

## Beschreibung

Die Erfindung betrifft eine Befestigungsvorrichtung zur Befestigung eines Abdeckmittels an einem C-Bogen-Röntgengerät als Arbeits- oder Untersuchungsgerät nach dem Oberbegriff des Anspruches 1.

Grundsätzlich ist es in vielen Arbeitsbereichen erforderlich, Arbeits- oder Untersuchungsgeräte abzudecken, um sie vor Verschmutzung zu schützen. Dies gilt umso mehr im medizinischen oder therapeutischen Bereich. Hier sollen Arbeits- und Untersuchungsgeräte für den Patientenkontakt steril gehalten werden. Dies ist nur möglich, indem diese Geräte mittels einer sterilen Abdeckung eingepackt werden.

Dies soll am Beispiel eines mobilen Röntgengerätes erläutert werden. Ein derartiges Röntgengerät wird bei vielen Operationen eingesetzt und weist eine Bogenform auf, so dass es an den Operationstisch so herangeführt werden kann, dass unter dem Operationstisch die Strahlenquelle sitzt, die durch Tisch und Patient hindurch nach oben strahlt, so dass in der oberen Bildgebertrommel ein Röntgenbild entsteht. Eine umgekehrte Anordnung ist ebenso denkbar. Da dieses Gerät im Operationssaal eingesetzt wird, sollte es steril sein, was aber bei diesem sogenannten C-Bogen-Röntgengerät eine nicht lösbare Aufgabe ist. Daher wird der C-Bogen bisher mit einer Sterilfolie abgedeckt, also mit einem Abdeckmittel, das insofern am mobilen Röntgengerät befestigt werden muss. Das Gerät muss beweglich und drehbar bleiben, insbesondere wird der C-Bogen in der Halterung gedreht und dabei sollte die Folie möglichst am C-Bogen, also am Gestell des Gerätes sicher fixiert bleiben.

Dafür gibt es nun viele Lösungen, wie z.B. eine Befestigung mit Klebeband, Klettband oder auch Kunststoffclips, die die Sterilfolie am Gestell des Geräts festclipsen. Diese Lösungen bereiten jedoch Probleme, da die Folie verrutscht und dadurch die Sterilität nicht gewährleistet ist. Werden Befestigungsvorrichtungen in Form von Bügeln eingesetzt, können diese vom Bogen abrutschen, da erschwerend hinzukommt, dass das C-Bogen-Röntgengerät eine äußerst glatte, meist lackierte Oberfläche aufweist.

Eine aus der DE 20 2015 104 257 U1 bekannte Befestigungsvorrichtung ist so ausgebildet, dass sie zumindest in einem Anlagebereich, in dem im Gebrauchszustand die Befestigungsvorrichtung am Abdeckmittel zur Anlage kommt und damit kraftschlüssig am Arbeits- oder Untersuchungsgerät anliegt, rutschhemmend ausgebildet ist. Gleichzeitig ist damit die Befestigung für die meistgebräuchlichen C-Bogen-Geräte verschiedener Hersteller universell verwendbar, so dass nicht für jeden Gerätetyp eine eigene Abdeckung erforderlich ist. In der konkreten Ausführungsform wird vorzugsweise an der als Klemmbügel ausgebildeten Befestigungsvorrichtung eine Stelle ausgespart, an der ein Stück PVC- oder Silicon-Schlauch aufgesteckt ist. Dieses Schlauchstück hat einen "Gummieffekt", so dass die Befestigungsvorrichtung und damit auch das Abdeckmittel gut und kraftschlüssig am Rahmen des Arbeits- oder Untersuchungsgerät haftet, so dass die Sterilität einerseits leicht hergestellt werden kann, andererseits zuverlässig gewährleistet ist.

Eine vergleichbare Lösung ist aus der US 2013/0025605 A1 für die Befestigung eines Abdeckmittels an einem C-Bogen-Gerät bekannt. Elastische Bügel übergreifen den C-Bogen, um dadurch das Abdeckmittel zu fixieren, und werden durch elastische Pads kraftschlüssig am C-Bogen festgelegt.

Aus der DE 19 58 905 A1 ist es bekannt, eine sterile Abdeckung an einem Bogen eines C-Bogen-Röntgengeräts mit Stahlklammern so zu befestigen, dass sie nicht in Richtung auf das Operationsfeld fallen.

Ausgehend von diesem Stand der Technik liegt der vorliegenden Erfindung die Aufgabe zugrunde, eine zuverlässige Befestigung für Abdeckmittel an C-Bogen-Röntgengeräten zu schaffen, die leicht anbringbar und dennoch gleichzeitig einen bestmöglichen Schutz gegen versehentliche Verlagerungen in einem Gebrauchszustand ermöglicht.

Diese Aufgabe wird durch eine Befestigungsvorrichtung mit den Merkmalen des Anspruches 1 gelöst. Vorteilhafte Weiterbildungen sind Gegenstand der abhängigen Patentansprüche. Die in den Patentansprüchen einzeln aufgeführten Merkmale sind in technologisch sinnvoller Weise miteinander kombinierbar und können durch erläuternde Sachverhalte aus der Beschreibung und durch Details aus den Figuren ergänzt werden, wobei weitere Ausführungsvarianten der Erfindung aufgezeigt werden.

Die Befestigungsvorrichtung umgreift das C-Bogen-Röntgengerät als Arbeits- oder Untersuchungsgerät zumindest bereichsweise mit wenigstens einem elastisch verformbaren Klemmbügel formschlüssig. Dabei ist zumindest ein Stopfen zum Herstellen des Anlagekontakts zerstörungsfrei reversibel form-, kraft- und/oder stoffschlüssig mit der übrigen Befestigungsvorrichtung verbunden oder verbindbar, wobei der zumindest eine Stopfen zumindest ein Fixiermittel aufweist und wobei zwischen dem Fixiermittel des Stopfens und der übrigen Befestigungsvorrichtung das Abdeckmittel fixierbar ist. Der Stopfen ist auf einer Innenfläche der Befestigungsvorrichtung angebracht bzw. dort montiert, wobei bei einer Montage des Stopfens gleichzeitig das Abdeckmittel fixiert wird. Das formschlüssige Umgreifen kann zusätzlich zu einer kraftschlüssigen Fixierung der Befestigungsvorrichtung erfolgen, beispielsweise durch eine Rastverbindung. Dadurch ist die Befestigungsvorrichtung leicht anbringbar und bietet dennoch einen bestmöglichen Schutz gegen versehentliche Verlagerungen. Besonders vorteilhaft kann die Befestigungsvorrichtung auch an vergleichsweise schmal ausgeführten C-Bogen-Röntgengeräten, die dadurch eine vergleichsweise kleine Anlagefläche aufweisen, sicher und zuverlässig fixiert werden. Zusätzlich zu dem gegebenenfalls herstellbaren Kraftschluss kann die Fixierung durch den Formschluss erfolgen. Die Stopfen können gegebenenfalls mehrfach verwendet werden und die Befestigungsvorrichtung kann zu Montagezwecken leicht mit dem Stopfen verbunden werden.

Die Stopfen können rutschhemmend ausgeführt sein. Die rutschhemmenden Stopfen können dabei ein gummiartiges Material aufweisen. Dies ermöglicht eine besonders sichere Fixierung der Befestigungsvorrichtung an dem C-Bogen-Röntgengerät ermöglichen. Das Fixiermittel kann dazu beispielsweise einen Bundkragen des Stopfens aufweisen. Das Abdeckmittel kann zwischen dem Bundkragen und der Innenseite angeordnet werden, beispielsweise dort kraftschlüssig und/oder formschlüssig fixiert werden. Zur formschlüssigen Fixierung kann das Abdeckmittel zumindest einen Durchbruch zur Aufnahme des Stopfens aufweisen. Der Durchbruch kann als Stanzung und/oder Loch in das Abdeckmittel eingebracht sein, wobei der Stopfen im montierten Zustand durch den Durchbruch hindurch ragt, sodass das Abdeckmittel dadurch gegen Verrutschen gesichert ist. Grundsätzlich kann das Abdeckmittel aber auch erst beim Anbringen des Stopfens vom Stopfen durchstanzt werden. Mittels des Bundkragens des Fixiermittels kann zusätzlich eine kraftschlüssige Fixierung und/oder eine Abdichtung erfolgen.

Vorzugsweise umgreift die Befestigungsvorrichtung im Gebrauchszustand den Geräterahmen des C-Bogen-Röntgengeräts auf mehr als drei Seiten und/oder mit einem Zentriwinkel von mehr als 200°. Dadurch ist eine zuverlässige Fixierung des Abdeckmittels am Geräterahmen sichergestellt und das Abdeckmittel gegen unbeabsichtigtes Lösen und Verrutschen weitestgehend gesichert.

Bevorzugt weist der zumindest eine Stopfen die mit der Anlagefläche des Arbeits- oder Untersuchungsgeräts in einen Anlagekontakt bringbare Fixierfläche auf. Die Fixierfläche kann in Form eines Bundkragens ausgeführt sein, wobei vorzugsweise eine Innenseite des Bundkragens wie vorab beschrieben zum Abdichten, kraftschlüssigen und/oder formschlüssigen Fixieren des Abdeckmittels an der Innenseite der Befestigungsvorrichtung und eine dieser gegenüberliegende Oberseite des Stopfens die Fixierfläche ausbildet. Der Stopfen kann dazu ein gummiartiges Material aufweisen. Die Fixierfläche ist im Gebrauchszustand der Anlagefläche des C-Bogen-Röntgengeräts zugewandt und steht gegebenenfalls mit dieser in einem rutschhemmenden Anlagekontakt. Dazu ist die Fixierfläche des Stopfens bevorzugt rutschhemmend ausgebildet, beispielsweise mittels des gummiartigen Materials.

Gemäß einem weiteren Ausführungsbeispiel kann der Klemmbügel C-förmig gestaltet sein. Dadurch kann einerseits eine leichte Montage durch Aufbiegen des Klemmbügels und andererseits ein Verrasten bzw. Hintergreifen des Klemmbügels mit einem Gestell des C-Bogen-Röntgengeräts erfolgen. Der Klemmbügel umgreift also das C-Bogen-Röntgengerät zumindest bereichsweise formschlüssig. Dadurch ist eine besonders sichere und zuverlässige Fixierung des Abdeckmittels an dem C-Bogen-Röntgengerät möglich.

Gemäß einer weiteren bevorzugten Alternative ist vorgesehen, dass der Klemmbügel zwei gegenüberliegend angeordnete und gebogene Enden aufweist, mittels denen das C-Bogen-Röntgengerät zumindest bereichsweise formschlüssig umgreifbar ist. Die gebogenen Enden bilden Rastvorsprünge, die im Gebrauchszustand mit dem C-Bogen-Röntgengerät verrastet sind.

Der Stopfen ist auf einer Innenseite des Klemmbügels angeordnet.

Dadurch kann dieser zwei Funktionen erfüllen, nämlich die Fixierung des Abdeckmittels an der übrigen Befestigungsvorrichtung, beziehungsweise an dem Klemmbügel der Befestigungsvorrichtung, und den rutschhemmenden Anlagekontakt der Fixierfläche des Stopfens an der Anlagefläche des C-Bogen-Röntgengeräts.

Es ist außerdem denkbar, dass die Innenseite des Klemmbügels zumindest einen Sitz zur Lagerung des Stopfens aufweist. Bei dem Sitz kann es sich um eine einfache Bohrung, insbesondere Sacklochbohrung handeln. Mittels der Bohrung kann durch Einstecken des Stopfens ein Presssitz hergestellt werden. Dadurch ist eine besonders einfache und gegebenenfalls auch reversible Montage des Stopfens und damit auch des Abdeckmittels an der übrigen Befestigungsvorrichtung beziehungsweise dem Klemmbügel der Befestigungsvorrichtung möglich. Es ist denkbar, den Klemmbügel und den zumindest einen Stopfen, vorzugsweise eine Vielzahl der Stopfen, mehrfach zu verwenden. Das eventuell unsteril gewordene Abdeckmittel kann also ausgetauscht werden. Es ist denkbar, dass der Stopfen bzw. die Vielzahl der Stopfen und der zumindest eine Klemmbügel autoklavierbar und/oder zumindest chemisch desinfizierbar sind.

Der zumindest eine Klemmbügel, vorzugsweise eine Vielzahl der Klemmbügel, ist bevorzugterweise in ihrer Form an eine Außenkontur des C-Bogen-Röntgengeräts, insbesondere an die Form seines Gestells angepasst. Insbesondere kann der Klemmbügel der Befestigungsvorrichtung in seiner Form an den Querschnitt des Geräterahmens angepasst sein. Dadurch kann eine noch bessere Fixierung erfolgen.

Mit der Befestigungsvorrichtung kann so auf einfache Art und Weise die Befestigung einer Abdeckfolie als das Abdeckmittel erfolgen.

Weitere Vorteile ergeben sich aus den Unteransprüchen und der nachfolgenden Beschreibung eines bevorzugten Ausführungsbeispiels.

Im Folgenden wird die Erfindung anhand eines in den Figuren dargestellten Ausführungsbeispiels näher erläutert. Es zeigen:
- Figur 1: eine schematische Seitenansicht eines nicht abgedeckten C-Bogen-Röntgengeräts als Arbeits- oder Untersuchungsgerät;
- Figur 2: eine Seitendetailansicht eines Klemmbügels und Stopfens zum Befestigen eines Abdeckmittels an einer Befestigungsvorrichtung zum Abdecken des in Figur 1 dargestellten C-Bogen-Röntgengeräts;
- Figur 3: einen schematischen Querschnitt durch ein Gestell des in Figur 1 gezeigten C-Bogen-Röntgengeräts, wobei dieses durch ein mit einer Befestigungsvorrichtung befestigtes Abdeckmittel abgedeckt ist, das in einen sehr engen Drehspalt eingreift;
- Figur 4: einzelne Elemente eines das Abdeckmittel aufweisenden kompletten Sterilbezugs für einen Geräterahmen des in Figur 1 gezeigten C-Bogen-Röntgengeräts;
- Figur 5: das in Figur 1 gezeigte C-Bogen-Röntgengerät zusammen mit dem in Figur 4 gezeigten Sterilbezug, wobei mittels Pfeilen angedeutet ist, wie dieser an dem Gerät anbringbar ist;
- Figur 6: die in Figur 1 gezeigte Seitenansicht des C-Bogen-Röntgengeräts, jedoch im mittels des in Figur 4 gezeigten Sterilbezugs abgedeckten Gebrauchszustand; und
- Figur 7: die in Figur 6 gezeigte Seitenansicht, wobei im Unterschied der Geräterahmen ohne Beeinträchtigung des Sterilbezugs verdreht ist.

### Beschreibung bevorzugter Ausführungsbeispiele

Die Erfindung wird jetzt beispielhaft unter Bezug auf die beigefügten Zeichnungen näher erläutert. Allerdings handelt es sich bei den Ausführungsbeispielen nur um Beispiele, die nicht das erfinderische Konzept auf eine bestimmte Anordnung beschränken sollen. Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Die Figuren zeigen eine Befestigungsvorrichtung 1 zur Befestigung eines Abdeckmittels 11 an einem C-Bogen-Röntgengerät 10 als Arbeits- oder Untersuchungsgerät. Derartige Abdeckmittel 11 in Form von meist Folien, Textilien, Tüchern oder Geweben und Kombinationen aus diesen Materialien müssen in verschiedensten Anwendungsbereichen an C-Bogen-Röntgengeräten 10 angebracht werden. Grundsätzlich sind derartige C-Bogen-Röntgengeräte 10 als Arbeits- oder Untersuchungsgeräte vor allem im medizinischen und therapeutischen Bereich insbesondere dann abzudecken, wenn diese Geräte nicht ohne weiteres sterilisierbar sind. Einsatzzwecke für die hier erläuterte Befestigungsvorrichtung ergeben sich damit überall dort, wo ein Abdeckmittel zuverlässig an einem rutschigen Untergrund festgelegt werden muss.

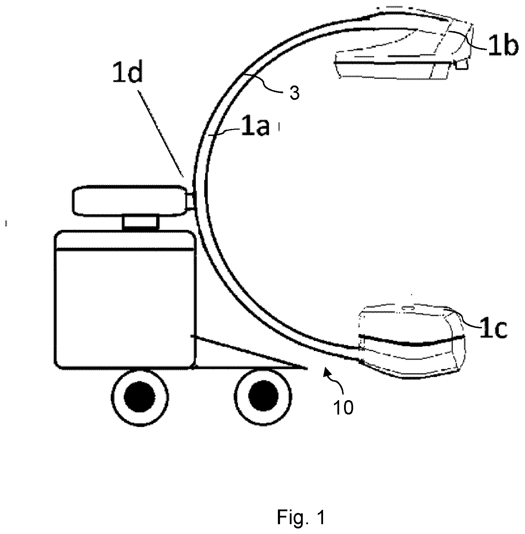

Im Rahmen dieser Anmeldung wird der erfindungsgemäße Einsatzbereich anhand eines C-Bogen-Röntgengeräts erläutert, das insbesondere in Operationssälen Verwendung findet. Figur 1 zeigt insofern ein mobiles Röntgengerät als Arbeits- oder Untersuchungsgerät mit einem Gestell beziehungsweise Geräterahmen 1a, der eine Bogenform hat. Für viele Operationen wird ein derartiges mobiles Röntgengerät eingesetzt, da es mit der Bogenform als C-Bogen-Röntgengerät 10 an den Operationstisch herangeführt werden kann. Am Operationstisch befindet sich vorzugsweise unter dem Tisch in einem Unterteil beziehungsweise Röntgengenerator 1c die Strahlenquelle, die durch Tisch und Patient hindurch nach oben strahlt, so dass in einem Oberteil beziehungsweise einer Kamera/Bildverstärker 1b in der oberen Bildgebertrommel ein Röntgenbild entsteht. Es versteht sich von selbst, dass vergleichbare Geräte, die in ähnlicher Weise abgedeckt werden müssen, ebenfalls mit einer derartigen Befestigungsvorrichtung versehen werden können, um Abdeckmittel allgemein und nicht nur Sterilfolien daran anzubringen.

Das nicht abgedeckte mobile C-Bogen-Röntgengerät 10 stellt im Operationssaal ein erhebliches Risiko als Keimüberträger dar, da es trotz glatter Oberflächen nicht vollständig steril zu bekommen ist. Aus diesem Grund wird es mit einer Sterilfolie abgedeckt, die nach der jeweiligen OP entsorgt werden kann. Damit nun das C-Bogen-Röntgengerät 10 beweglich und drehbar bleiben kann, muss das Abdeckmittel 11 möglichst eng an dem Gestell beziehungsweise Geräterahmen 1a, also an dem Bogen anliegen. Erfolgt eine Befestigung z.B. mit Kunststoffclips, führt dies dazu, dass diese Bügel leicht vom Bogen oder Abdeckmittel abrutschen, da die Oberfläche des Geräterahmens 1a rutschig, meist lackiert ist. Die kraftschlüssige Verbindung zwischen Befestigungsvorrichtung 1 und Arbeits- oder Untersuchungsgerät 10 reicht nicht aus, um das Abdeckmittel 11 am Geräterahmen 1a zu halten.

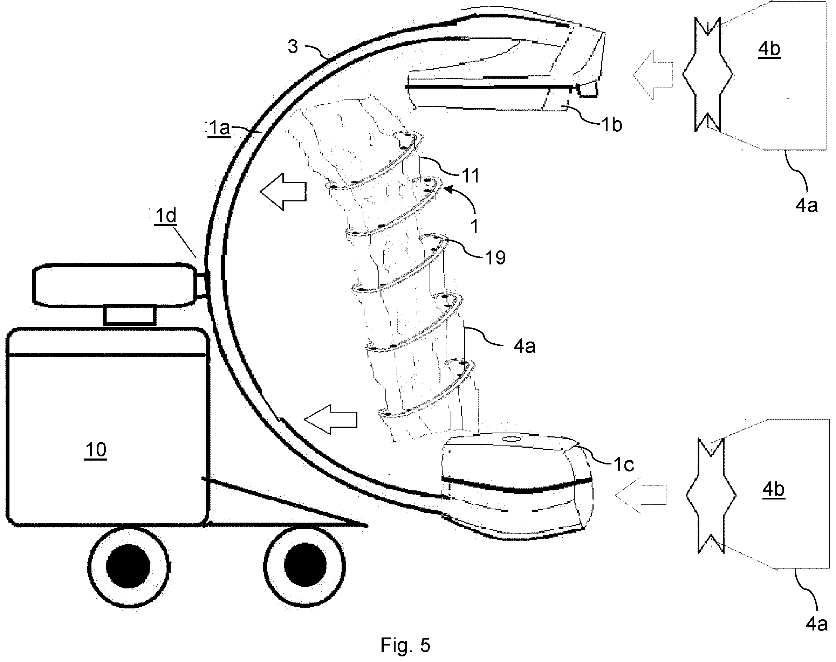

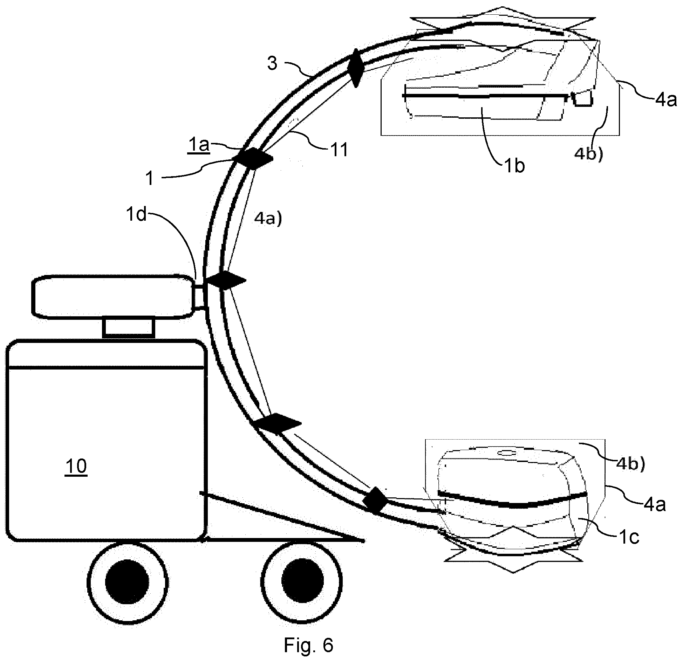

Die Figuren 5 und 6 zeigen die Befestigung des Abdeckmittels 11 am C-Bogen-Röntgengerät 10. Das Abdeckmittel 11 besteht aus mehreren Teilen, wobei ein Endabschnitt 4b mit dem Abdeckmittel 11 vorzugsweise verbunden ist. Der Endabschnitt 4b wird in Fig. 5 über die Kamera 1b und den Röntgengenerator 1c gestülpt und das Abdeckmittel 11 wird am Geräterahmen 1a befestigt. Aus Figur 5 wird ersichtlich, dass es sich bei dem Abdeckmittel 11 vorzugsweise um ein Folienflachstück handelt, an dem mehrere der Befestigungsvorrichtungen 1 bereits angebracht sind. Wird also der Endabschnitt 4b über die Kamera 1b geführt, können die vorzugsweises bereits an den Geräterahmen 1a ihrer Form nach angepassten Befestigungsvorrichtungen 1 am Geräterahmen 1a festgelegt werden. Bei diesen Befestigungsvorrichtungen handelt es sich um wenigstens einen elastisch verformbaren Klemmbügel.

Sobald der Geräterahmen 1a mit dem Abdeckmittel 11 abgedeckt ist, endet das Folienschlauchstück des Abdeckmittels 11 etwa im Bereich des Röntgengenerators 1c. Dort wird der weitere Endabschnitt 4b über den Röntgengenerator 1c übergestülpt, so dass damit auch der Endbereich des Abdeckmittels 11 gesichert wird. Dies führt zu einem "eingepackten" C-Bogen-Röntgengerät gemäß Fig. 6.

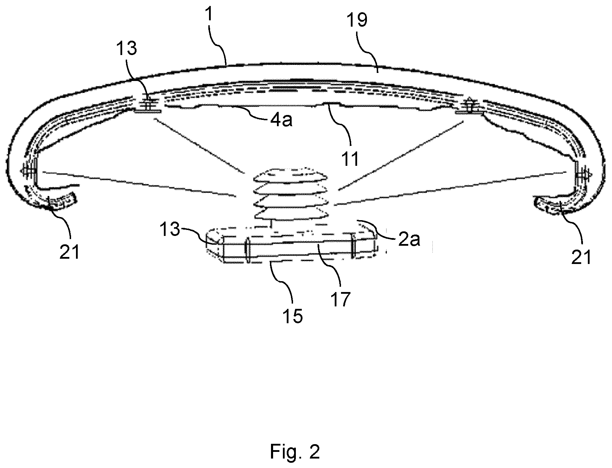

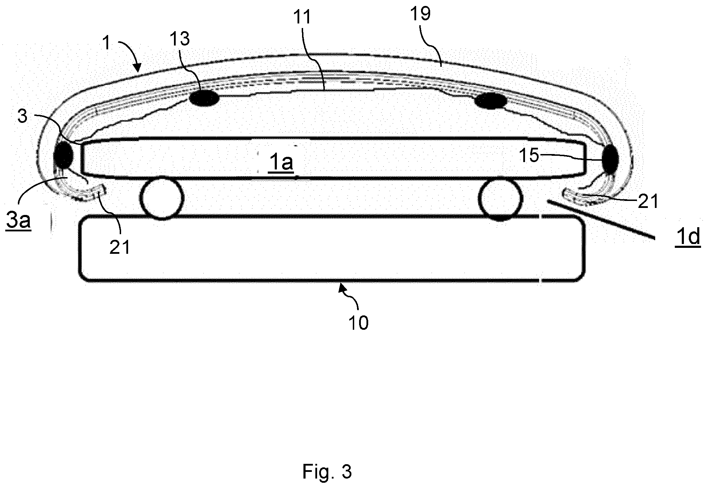

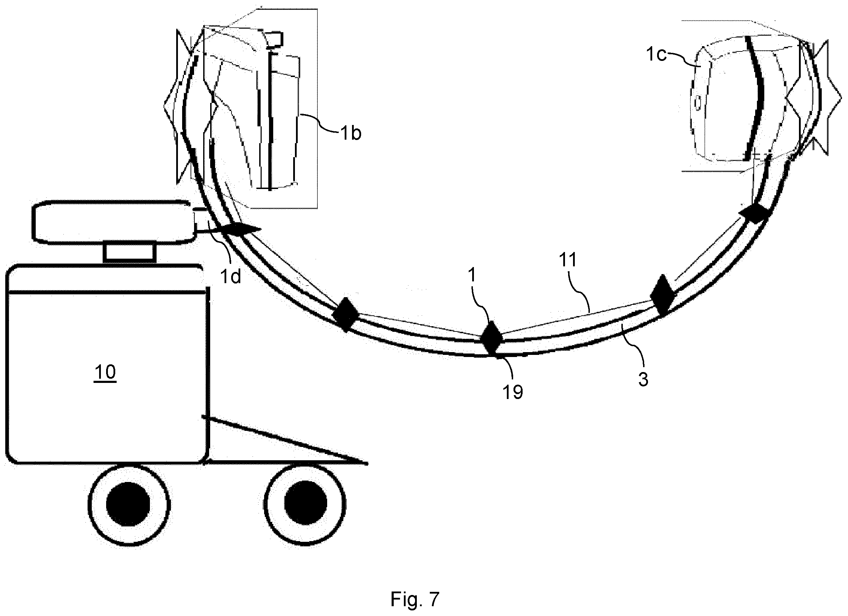

Im Ausführungsbeispiel ist die Befestigungsvorrichtung 1 ein elastisch formbarer Klemmbügel 19, wie er in den Fig. 2 und 3 dargestellt ist. Es ist zu erkennen, dass der Klemmbügel 19 C-förmig gestaltet ist und den Geräterahmen 1a zumindest teilweise formschlüssig hintergreift bzw. mit diesem verrastet ist. Genauer umgreifen die Klemmbügel 19 der Befestigungsvorrichtung 1 im Gebrauchszustand den Geräterahmen 1a des C-Bogen-Röntgengeräts 10 auf mehr als drei Seiten und/oder mit einem Zentriwinkel von mehr als 200°. Dadurch liegen diese Klemmbügel so am Geräterahmen an, dass die gebogenen Enden 21 des Klemmbügels 19, die Rastvorsprünge bilden, auch bei Bewegung des Geräterahmens durch den Drehspalt 1d gelangen können. Der Drehspalt 1d verbleibt zwischen einer Antriebsvorrichtung zum Verdrehen des Geräterahmens 1a und des Geräterahmens 1a selbst. Der Drehspalt 1d kann vergleichsweise schmal ausgeführt sein, wobei dennoch vorteilhaft die Enden 21 kollisionsfrei in diesen eingreifen können, sodass der halbkreisförmig gekrümmte Geräterahmen 1a auch mit montierten Befestigungsvorrichtungen 1, wie in den Figuren 6 und 7 illustriert, problemlos verdreht werden kann. Die Funktion des C-Bogen-Röntgengeräts 10 ist also auch in abgedecktem Zustand vollständig gewährleistet.

Figur 2 zeigt in einer dreidimensionalen Detailansicht einen Stopfen 13 mit einer Fixierfläche 15 und einem Fixiermittel 17. Das Fixiermittel 17 ist als rechteckiger Bundkragen des Stopfens 13 ausgestaltet, der sich am Ende eines Schaftes des Stopfens 13 befindet. Der Schaft des Stopfens 13 kann in einer in den Figuren nicht näher dargestellte Ausnehmung des Klemmbügels 19 angebracht werden. Bei der Ausnehmung kann es sich um eine Sacklochbohrung handeln, wobei der Schaft des Stopfens 13 dort mittels eines Presssitzes einbringbar ist. Die Ausnehmung bildet also einen Sitz für den Stopfen 13. Der Schaft kann, wie in Figur 2 zu erkennen, elastische Widerhaken bildende umlaufende Rippen aufweisen, die zusätzlich eine Verankerung in dem Sitz verbessern können.

Wie in Figur 3 zu erkennen, weist jeder der Klemmbügel 19 eine Vielzahl der Stopfen 13 auf, die an einer Innenseite des jeweiligen Klemmbügels 19 angebracht sind. Der Bundkragen bzw. das Fixiermittel 17 des Stopfens 13 dient zum kraftschlüssigen und formschlüssigen Fixieren des Abdeckmittels 11 an der Innenseite des Klemmbügels 19. Dazu kann das Abdeckmittel 11, das vorzugsweise ein flexibles Material 4a wie beispielsweise eine Folie umfasst, einen in den Figuren nicht näher dargestellten Durchbruch, beispielsweise eine Stanzung aufweisen. Der Durchbruch wird im montierten Zustand von dem Schaft des Stopfens durchgriffen. Der Durchbruch kann aber auch erst beim Fixieren des Stopfens erzeugt werden.

Eine Oberseite des Bundkragens bzw. des Fixiermittels 17 des Stopfens 13 weist die Fixierfläche 15 auf. Die Fixierfläche 15 ist im in Figur 3 gezeigten montierten Zustand dem Geräterahmen 1a des C-Bogen-Röntgengeräts 10 zugewandt. Genauer sind die Fixierflächen 15 der jeweiligen Stopfen 13 in dem Gebrauchszustand in einem rutschhemmenden Anlagekontakt mit einer seitlichen Anlagefläche 3 des Geräterahmens 1a.

Das Fixiermittel 17 des Stopfens 13 bildet außerdem eine Auflage 2a, die an der Innenseite des Klemmbügels 19 anlegbar ist. Das Abdeckmittel 11 bzw. das flexible Material 4a des Abdeckmittels 11 kann zwischen der Auflage 2a und der Innenseite des Klemmbügels 19 eingeklemmt und dort sicher fixiert werden.

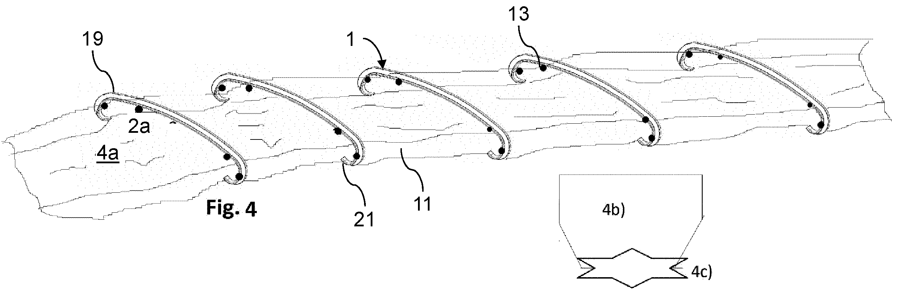

Figur 4 zeigt eine schematische dreidimensionale Ansicht eines kompletten Sterilbezugs der in den Figuren 3, 6 und 7 in montiertem Zustand zusammen mit dem Arbeits- oder Untersuchungsgerät 10 dargestellt ist. Es ist zu erkennen, dass das flexible Material 4a mit einer Vielzahl der Befestigungsvorrichtungen 1 bzw. Klemmbügel 19 verbunden ist. Es ist optional möglich, das flexible Material 4a durch ein Entfernen der jeweiligen Stopfen 13 von den jeweiligen Befestigungsvorrichtungen 1 wieder zu entfernen und durch ein neues flexibles Material zu ersetzen. Die Stopfen 13 und die Klemmbügel 19 sind also optional mehrfach verwendbar.

Wie in den Figuren 6 und 7 zu erkennen, kann der Geräterahmen 1a trotz des in Figur 4 dargestellten Sterilbezugs in montiertem Zustand ungehindert verdreht werden.

Das in den Figuren dargestellte C-Bogen-Röntgengerät 10 weist einen vergleichsweise dünnen Geräterahmen 1a für die die Aufnahme von Kamera/Bildverstärker bzw. Oberteil 1b und Röntgengenerator bzw. Unterteil 1c auf. Hierfür wurde nun eine spezielle in der Figur 2 dargestellte Klemmhalterung mittels des Stopfens 13 bereitgestellt, die den besonderen Anforderungen des im Querschnitt vergleichsweise kleinen Geräterahmens 1a entspricht.

Zusammenfassend umgreift die Befestigungsvorrichtung 1 den wesentlich dünneren Geräterahmen 1a zumindest bereichsweise formschlüssig. Der in Figur 4 dargestellte Sterilbezug kann mit den Klemmbügeln 19 der Befestigungsvorrichtung 1 an dem Geräterahmen 1a befestigt werden, wobei es möglich ist, die Klemmbügel 19 zu fixieren, ohne die Mimik zum Verstellen des Geräterahmens 1a zu behindern. Die Klemmbügel 19 der Befestigungsvorrichtung 1 sind dazu an den Geräterahmen 1a angepasst. Insbesondere bilden die Enden 21 der Klemmbügel 19 die vorab beschriebenen Rastvorsprünge, mittels denen die Klemmbügel 19 an dem Geräterahmen 1a verrastbar sind, was in Figur 3 gezeigt ist. Vorzugsweise ist der Klemmbügel 19 in seiner Form an den Querschnitt des Geräterahmens 1a angepasst.

Gleichzeitig können die Klemmbügel 19 gegen ein Verrutschen gesichert werden. Dies geschieht durch die Stopfen 13, genauer die gummiartige Fixierfläche 15 des Stopfens 13 an einer Innenseite der Klemmbügel 19. Ein entsprechender rutschhemmender Anlagekontakt zwischen den jeweiligen Fixierflächen 15 der Stopfen 13 und der Anlagefläche 3 des Arbeits- oder Untersuchungsgeräts 10 ist in Figur 3 zu erkennen. Die Auflagen 2a der Fixiermittel 17 sind als Bundkragen der Stopfen 13 ausgelegt, wobei die Stopfen 13 in Ausnehmungen der Klemmbügel 19 eingedrückt werden können. Dabei halten diese mittels der Auflage 2a gleichzeitig das flexible Material 4a fest, das der sterilen Abdeckung des bogenförmigen Geräterahmens 1a dient. Die Stopfen 13 können ein beliebiges, elastisches, gummiartiges und/oder rutschhemmendes Material, beispielsweise thermoplastisches Polyurethan (TPU), thermoplastisches Elastomer (TPE), und/oder Silikon aufweisen. Dadurch können vorteilhaft die rutschfeste Auflage 2a, die rutschfeste Fixierfläche 15 der Befestigungsvorrichtung 1 und/oder der Presssitz des Stopfens im Klemmbügel 19 bereitgestellt werden.

Die Befestigungsvorrichtung 1 bzw. der Klemmbügel 19 ist in ihrer/seiner Form an die Form des C-Bogen-Röntgengeräts 10 und insbesondere an die Form des Geräterahmens 1a angepasst. Grundsätzlich dient die Befestigungsvorrichtung 1 zur Befestigung einer Abdeckfolie als Abdeckmittel 11, also das flexible Material 4a, wenngleich auch andere Materialien durch eine derartige Befestigungsvorrichtung 1 festgelegt werden können. Wenngleich verschiedenste Einsatzbereiche möglich sind, ist der erfindungsgemäße Einsatzbereich die Befestigung einer Abdeckfolie als Abdeckmittel 11 an einem C-Bogen Röntgengerät 10 als Arbeits- oder Untersuchungsgerät.

### Bezugszeichenliste

- 1: Befestigungsvorrichtung
- 1a: Geräterahmen
- 1b: Kamera/Bildverstärker
- 1c: Röntgengenerator
- 1d: Drehspalt
- 2a: Auflage
- 3: Anlagefläche
- 4a: flexibles Material
- 4b: Endabschnitt
- 10: C-Bogen-Röntgengerät
- 11: Abdeckmittel
- 13: Stopfen
- 15: Fixierfläche
- 17: Fixiermittel
- 19: Klemmbügel
- 21: Enden

## Patentansprüche

1. Befestigungsvorrichtung (1), eingerichtet zur Befestigung eines Abdeckmittels (11) an einem C-Bogen-Röntgengerät (10) als Arbeits- oder Untersuchungsgerät, mit zumindest einer Fixierfläche (15), die mit zumindest einer Anlagefläche (3) des C-Bogen-Röntgengeräts (10) in einen fixierenden Anlagekontakt bringbar ist, wobei das Abdeckmittel (11) das C-Bogen-Röntgengerät (10) in einem Gebrauchszustand zumindest bereichsweise abdeckt und mittels der Befestigungsvorrichtung (1) fixiert ist, die wenigstens einen elastisch verformbaren Klemmbügel (19) aufweist,
wobei der Klemmbügel (19) das C-Bogen-Röntgengerät (10) zumindest bereichsweise formschlüssig umgreift,
wobei zumindest ein Stopfen (13) zum Herstellen des Anlagekontakts zerstörungsfrei reversibel form-, kraft- und/oder stoffschlüssig mit der übrigen Befestigungsvorrichtung (1) verbunden oder verbindbar ist und wobei der Stopfen (13) auf einer Innenseite des Klemmbügels (19) angeordnet ist,
**dadurch gekennzeichnet,**
**dass** der zumindest eine Stopfen (13) zumindest ein Fixiermittel (17) aufweist, wobei zwischen dem Fixiermittel (17) des Stopfens (13) und der übrigen Befestigungsvorrichtung (1) das Abdeckmittel (11) fixierbar ist.

2. Befestigungsvorrichtung nach Anspruch 1, **dadurch gekennzeichnet, dass** die Befestigungsvorrichtung im Gebrauchszustand einen Geräterahmen (1a) des C-Bogen-Röntgengeräts (10) auf mehr als drei Seiten und/oder mit einem Zentriwinkel von mehr als 200° umgreift.

3. Befestigungsvorrichtung nach Anspruch1 oder 2, **dadurch gekennzeichnet, dass** der zumindest eine Stopfen (13) rutschhemmend ausgeführt ist.

4. Befestigungsvorrichtung nach einem der vorhergehenden Ansprüche, **dadurch gekennzeichnet, dass** das Abdeckmittel (11) zumindest einen Durchbruch zur Aufnahme des Stopfens (13) aufweist.

5. Befestigungsvorrichtung nach einem der vorhergehenden Ansprüche, **dadurch gekennzeichnet, dass** der zumindest eine Stopfen (13) die mit der Anlagefläche (3) des C-Bogen-Röntgengeräts (10) in einen Anlagekontakt bringbare Fixierfläche (15) aufweist.

6. Befestigungsvorrichtung nach Anspruch 5, **dadurch gekennzeichnet, dass** die Fixierfläche (15) des Stopfens (13) rutschhemmend ausgebildet ist.

7. Befestigungsvorrichtung nach einem der vorhergehenden Ansprüche, **dadurch gekennzeichnet, dass** der Klemmbügel (19) C-förmig ausgebildet ist.

8. Befestigungsvorrichtung nach einem der vorhergehenden Ansprüche, **dadurch gekennzeichnet, dass** der Klemmbügel (19) zwei gegenüberliegend angeordnete und gebogene Enden (21) aufweist, mittels denen das C-Bogen-Röntgengerät zumindest bereichsweise formschlüssig umgreifbar ist.

9. Befestigungsvorrichtung nach einem der vorhergehenden Ansprüche, **dadurch gekennzeichnet, dass** die Innenseite des Klemmbügels (19) zumindest einen Sitz zur Lagerung des Stopfens (13) aufweist.

10. Befestigungsvorrichtung nach einem der vorhergehenden Ansprüche, **dadurch gekennzeichnet, dass** die Befestigungsvorrichtung (1) in ihrer Form an die Form des C-Bogen-Röntgengeräts (10), insbesondere an die Form des Geräterahmens (1a) angepasst ist.

11. Befestigungsvorrichtung nach Anspruch 10, **dadurch gekennzeichnet, dass** der Klemmbügel (19) der Befestigungsvorrichtung (1) in seiner Form an den Querschnitt des Geräterahmens (1a) angepasst ist

12. Befestigungsvorrichtung nach einem der vorhergehenden Ansprüche, **dadurch gekennzeichnet, dass** die Befestigungsvorrichtung zur Befestigung einer Abdeckfolie als Abdeckmittel (11) vorgesehen ist.

## Claims

1. A fastening device (1) configured for fastening a covering element (11) to a C-arm X-ray machine (10) as a work or examination device, comprising at least one fixing face (15), which can be brought into fixing bearing contact with at least one contact face (3) of the C-arm X-ray machine (10), wherein the covering element (11) covers the C-arm X-ray machine (10) at least in regions, when in use, and is fixed by means of the fastening device (1), which has at least one elastically deformable clamping bracket (19),
wherein the clamping bracket (19) engages around the C-arm X-ray machine (10) form-fittingly, at least in regions,
wherein at least one stopper (13) is non-destructively reversibly connected or connectable in a positive-locking, force-locking and/or integrally bonded manner to the rest of the fastening device (1) to establish the bearing contact, and wherein the stopper (13) is arranged on an inner side of the clamping bracket (19),
**characterised in that**
the at least one stopper (13) comprises at least one fixing means (17), wherein the covering element (11) is fixable between the fixing means (17) of the stopper (13) and the rest of the fastening device (1).

2. A fastening device in accordance with claim 1, **characterised in that** the fastening device, when in use, engages around a machine frame (1a) of the C-arm X-ray machine (10) on more than three sides and/or with a central angle of more than 200°.

3. A fastening device in accordance with claim 1 or 2, **characterised in that** the at least one stopper (13) is embodied in a non-slip manner.

4. A fastening device in accordance with one of the preceding claims, **characterised in that** the covering element (11) has at least one aperture for receiving the stopper (13).

5. A fastening device in accordance with one of the preceding claims, **characterised in that** the at least one stopper (13) comprises the fixing face (15) which can be brought into bearing contact with the contact face (3) of the C-arm X-ray machine (10).

6. A fastening device in accordance with claim 5, **characterised in that** the fixing face (15) of the stopper is configured in a non-slip manner.

7. A fastening device in accordance with one of the preceding claims, **characterised in that** the clamping bracket (19) is C-shaped.

8. A fastening device in accordance with one of the preceding claims, **characterised in that** the clamping bracket (19) has two oppositely arranged and bent ends (21), by means of which the C-arm X-ray machine can be engaged around in a positive-locking manner at least in regions.

9. A fastening device in accordance with one of the preceding claims, **characterised in that** the inner side of the clamping bracket (19) comprises at least one seat for supporting the stopper (13).

10. A fastening device in accordance with one the preceding claims, **characterised in that** the fastening device (1) is adapted in its shape to the shape of the C-arm X-ray machine (10), in particular to the shape of the machine frame (1a).

11. A fastening device in accordance with claim 10, **characterised in that** the clamping bracket (19) of the fastening device (1) is adapted in its shape to the cross section of the machine frame (1a).

12. A fastening device in accordance with one of the preceding claims, **characterised in that** the fastening device is provided for fastening a covering film as covering element (11).

## Revendications

1. Dispositif de fixation (1), prévu pour la fixation d'un moyen de couverture (11) sur un appareil à rayons X incurvé en C (10) en tant qu'appareil de travail ou d'examen, comportant au moins une face de fixation (15) qui peut être mise en contact d'installation de fixation avec au moins une face d'installation (3) de l'appareil à rayons X incurvé en C (10), dans lequel le moyen de fixation (11) couvre au moins par zones l'appareil à rayons X incurvé en C (10) dans un mode d'utilisation et est fixé au moyen du dispositif de fixation (1), lequel comprend au moins un serre-câble (19) déformable élastiquement,
le serre-câble (19) enserrant par complémentarité de forme l'appareil à rayons X incurvé en C (10) au moins par zones,
au moins un bouchon (13) pour la réalisation du contact d'installation étant relié ou reliable au reste du dispositif de fixation (1) de manière réversible non destructible par complémentarité de forme, de force et/ou de matière et le bouchon (13) étant disposé sur un côté interne du serre-câble (19),
**caractérisé en ce que**
le bouchon (13) présente au moins un moyen de fixation (17), le moyen de fermeture (11) pouvant être fixé entre le moyen de fixation (17) du bouchon (13) et le reste du dispositif de fixation (1).

2. Dispositif de fixation selon la revendication 1, **caractérisé en ce que** le dispositif de fixation enserre en mode d'utilisation un cadre d'appareil (1a) de l'appareil à rayons X incurvé en C (10) sur plus de trois côtés et/ou avec un angle de centrage de plus de 200 degrés.

3. Dispositif de fixation selon la revendication 1 ou 2, **caractérisé en ce que** le bouchon (13) est réalisé anti-dérapant.

4. Dispositif de fixation selon l'une des revendications précédentes, **caractérisé en ce que** le moyen de fermeture (11) présente au moins un perçage pour loger le bouchon (13).

5. Dispositif de fixation selon l'une des revendications précédentes, **caractérisé en ce que** le bouchon (13) présente la face de fixation qui peut être mise en contact d'installation avec la face d'installation (3) de l'appareil à rayons X incurvé en C (10).

6. Dispositif de fixation selon la revendication 5, **caractérisé en ce que** la face de fixation (15) du bouchon (13) est réalisée anti-dérapante.

7. Dispositif de fixation selon l'une des revendications précédentes, **caractérisé en ce que** le serre-câble (19) est en forme de C.

8. Dispositif de fixation selon l'une des revendications précédentes, **caractérisé en ce que** le serre-câble (19) présente deux extrémités (21) disposées face à face et incurvées, au moyen desquelles l'appareil à rayons X incurvé en C peut être enserré au moins par endroits par complémentarité de forme.

9. Dispositif de fixation selon l'une des revendications précédentes, **caractérisé en ce que** le côté intérieur du serre-câble (19) présente au moins un logement pour loger le bouchon (13).

10. Dispositif de fixation selon l'une des revendications précédentes, **caractérisé en ce que** le dispositif de fixation (1) a une forme adaptée à la forme de l'appareil à rayons X incurvé en C, en particulier à la forme du cadre de l'appareil (1a).

11. Dispositif de fixation selon la revendication 10, **caractérisé en ce que** le serre-câble (19) du dispositif de fixation (1) a une forme adaptée à la section transversale du cadre de l'appareil (1a).

12. Dispositif de fixation selon l'une des revendications précédentes, **caractérisé en ce que** le dispositif de fixation est prévu pour la fixation d'une feuille de couverture en tant que moyen de couverture (11).
